# EUROPÄISCHE PATENTSCHRIFT

(11) **EP 1 152 776 B1**
(45) Veröffentlichungstag und Bekanntmachung des Hinweises auf die Patenterteilung: **22.05.2002**
(21) Anmeldenummer: 00914033.6
(22) Anmeldetag: 11.02.2000
(51) Int. Cl.: A61L 27/18, A61L 27/50, A61L 27/54

(54) **BIOABBAUBARE, INJIZIERBARE OLIGOMER-POLYMER-ZUSAMMENSETZUNG**
BIODEGRADABLE, INJECTABLE OLIGOMER-POLYMER COMPOSITION
COMPOSITION OLIGOMERE-POLYMERE INJECTABLE BIODEGRADABLE

(30) Priorität: 20.02.1999 DE 19908753
(43) Veröffentlichungstag der Anmeldung: 14.11.2001
(73) Patentinhaber: Jenapharm GmbH & Co. KG, 07745 Jena (DE)
(72) Erfinder: VÖLKEL, Christoph, D-07743 Jena (DE); PFEIFFER, Manuela, D-07745 Jena (DE); FRICKE, Sabine, D-00749 Jena (DE); VOGT, Sebastian, D-07749 Jena (DE); BORMANN, Ernst-Joachim, D-07747 Jena (DE); SCHNABELREUTH, Matthias, D-07745 Jena (DE); BEER, Brigitte, D-07751 Zöllnitz (DE)
(74) Vertreter: Cramer, Eva-Maria
(86) Internationale Anmeldenummer: DE0000444
(87) Internationale Veröffentlichungsnummer: WO0048643

(56) Entgegenhaltungen:
- EP-A- 0 275 961
- EP-A- 0 544 097
- US-A- 4 938 763
- US-A- 5 702 717

## Beschreibung

Die vorliegende Erfindung betrifft eine bioabbaubare, injizierbare Oligomer-Polymer-Zusammensetzung bestehend aus einer Kombination von mindestens zwei biologisch abbaubaren Hilfsstoffen und mindestens einem biologisch aktiven Wirkstoff, wobei die biologisch abbaubaren Hilfsstoffe oligomere und polymere Ester von Hydroxycarbonsäuren sind.

Implantate zur Applikation biologisch aktiver Stoffe können durch Verpressen unter aseptischen Bedingungen hergestellt werden. Diese Implantate besitzen den Nachteil, daß sie sich nicht den räumlichen Gegebenheiten des Applikationsortes anzupassen vermögen und dort Druck- bzw. Schmerzgefühl nach Applikation hervorrufen.

Eine bekannte Lösung zur Herstellung injizierbarer Implantate besteht ferner in der Verwendung von Mikrokapseln. Die gebräuchlichsten Herstellungsverfahren für Mikrokapseln bzw. Mikrospheren sind die sogenannte "Solvent Evaporation Technique" die "Spray Drying Technique" oder die "Double Emulsion Technique". Diese Verfahren erfordern organische Lösemittel oder Lösemittelgemische wie beispielsweise Dichlormethan, Trichlormethan oder Dichlormethan/Methanol, die für den lebenden Organismus toxisch oder zumindest physiologisch bedenklich sind. Ein gemeinsamer Nachteil dieser Verfahren ist daher der Restlösemittelgehalt. Die "Spray Drying Method" ist darüber hinaus mit einem relativ hohen apparativen Aufwand verbunden.

Es besteht weiterhin die Möglichkeit, aus einer Lösung oder Suspension mit biologisch aktivem Stoff nach parenteraler Applikation ein in situ Implantat zu erzeugen.

Die in situ Bildung eines Implantats kann auf unterschiedliche Weise induziert werden.

In der US-PS 4,938,763 wird die biologisch aktive Substanz in einer Lösung des bioabbaubaren Polymers, z. B. einem Polylactid, gelöst oder dispergiert und diese Lösung oder Dispersion injiziert. Nach dem Injizieren bildet sich bei Kontakt mit der Körperflüssigkeit ein festes Implantat, bestehend aus dem gefällten oder koagulierten bioabbaubaren Polymer und der biologisch aktiven Substanz aus. Das Lösungsmittel wandert dabei aus dem Implantat heraus und verteilt sich im Organismus. Ein Nachteil dieser Methode besteht darin, daß die verwendeten Lösungsmittel, wie z. B. N-Methyl-2-pyrrolidon, physiologisch wirksam und daher nicht oder nur in geringem Umfang parenteral applizierbar sind.

In der genannten und weiteren Patentschriften (US-PS 5,278,201; US-PS 5,278,202) werden ferner als in situ Implantatmaterialien flüssige Acrylat-terminierte Prepolymere vorgeschlagen, die beispielsweise durch Umsetzung von Poly(D,L-lactid-co-ε-caprolacton) mit reaktiven Acrylsäurederivaten hergestellt werden können. Die Injektion des flüssigen Prepolymers erfolgt im Gemisch mit der biologisch aktiven Substanz und einem geeigneten Initiator (z. B. Dibenzoylperoxid), der die Aushärtung des Prepolymers und damit die Implantatbildung im Körper auslöst. Die Nachteile dieser Methode liegen in einem erheblich höheren Synthese- und Reinigungsaufwand zur Bereitstellung der biologisch abbaubaren Polymere und in der parenteralen Verabreichung eines physiologisch nicht unbedenklichen Radikalbildners als Initiator. Außerdem existieren bisher keine Erfahrungen zur Bioverträglichkeit der genannten Acrylat-terminierten Prepolymere und zur Biodegradation der aus diesen Substanzen gebildeten Implantate.

In der US-PS 5,702,71 7 werden Block-Copolymere aus Polyethylenglykol und Polylactid bzw. Polycaprolacton beschrieben, die in wäßriger Lösung bei Raumtemperatur als injizierbare Flüssigkeit vorliegen und bei Körpertemperatur ein Gel bilden, das den biologisch aktiven Stoff enthält. Ein Nachteil dieser Variante besteht darin, daß die Temperatur für den Sol-Gel-Übergang von einer Vielzahl unterschiedlicher Parameter abhängig ist, wie z. B. von der Zusammensetzung und dem Polymerisationsgrad der einzelnen Blöcke im Block-Copolymer, dem Molekulargewicht des Block-Copolymers sowie der Polymerkonzentration in der wäßrigen Lösung.

Die Patentschriften EP 0 544 097 A und EP 0 275 961 A offenbaren feste bzw. halbfeste Oligomer-Polymer-Zusammensetzungen aus biologisch abbaubaren Hydroxycarbonsäureesteren, die biologisch aktive Wirkstoffe enthalten.

Der vorliegenden Erfindung liegt die Aufgabe zugrunde, eine Oligomer-Polymer-Zusammensetzung als injizierbares Implantat zur Verfügung zu stellen, welches die Nachteile des Standes der Technik überwindet.

Die Aufgabe wird erfindungsgemäß dadurch gelöst, daß eine injizierbare Oligomer-Polymer-Zusammensetzung bestehend aus einer Kombination von mindestens zwei biologisch abbaubaren Hilfsstoffen und mindestens einem biologisch aktiven Wirkstoff zur Verfügung gestellt wird, wobei die biologisch abbaubaren Hilfsstoffe Polymerisationsprodukte von gleichen oder unterschiedlichen Hydroxycarbonsäuren sind.

Besonders bevorzugt ist es, daß die Hydroxycarbonsäuren Milchsäure oder Glycolsäure sind.

Erfindungsgemäß bevorzugt ist ferner, daß jeweils mindestens einer der biologisch abbaubaren Hilfstoffe ein flüssiges niedermolekulares Oligomer und der andere ein festes höhermolekulares Polymer ist.

Weiterhin ist erfindungsgemäß bevorzugt, daß das flüssige niedermolekulare Oligomer eine Verbindung der allgemeinen Formel I, II oder III 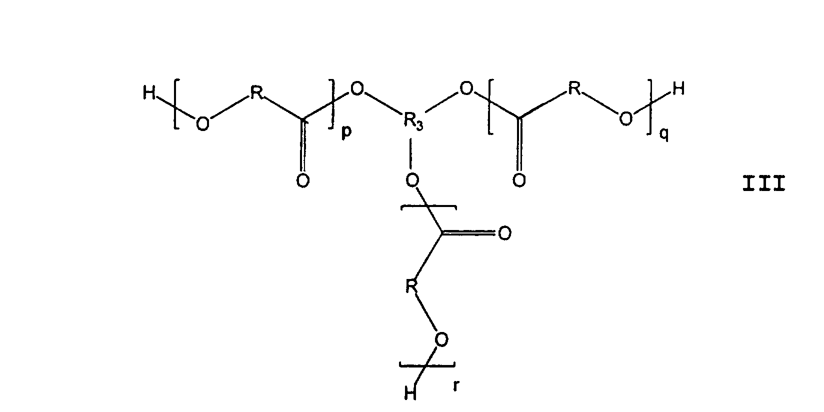 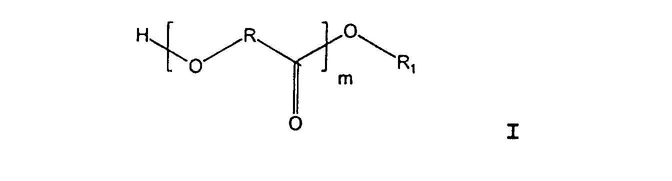 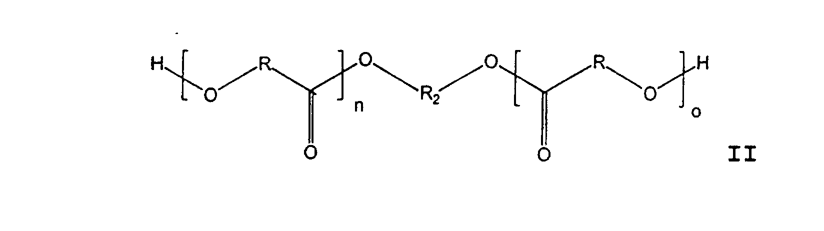 ist, worin
R für die Variablen m, n, o ,p, q und r jeweils gleich oder unterschiedlich ist und für -CH₂-, -CH(CH₃)-, - (CH₂)₅-, -CH₂-CH₂-O-CH₂-, -CH₂-CH₂-O-CH₂-CH₂-O-CH₂- oder deren Homologe mit jeweils bis zu 5 weiteren C-Atomen steht,
R₁ für -CH₂-COOY, -CH(CH₃)-COOY, -CH₂-CH₂-COOY, -CH₂-CH₂-CH₂-COOY, -CH₂-CH₂-CH₂-CH₂-COOY, -CH₂-CH₂-CH₂-CH₂-CH₂-COOY, -CH₂-CH (CH₃)-Y, - (cyclo-C₆H₁₁) oder -CH₂-C₆H₅- steht,
R₂ für -CH₂-CH(CH₃)-, -CH₂-CH₂-CH₂-, -CH₂-CH₂-CH₂-CH₂-, -CH₂-CH₂-CH₂-CH₂-CH₂-, -(CH₂)₂-O-(CH₂)₂-O-(CH₂)₂-, - (CH₂)₂-O-(CH₂)₂-O-(CH₂)₂-O-(CH₂)₂-, - (CH₂)₂-O-(CH₂)₂-O-(CH₂)₂-O-(CH₂)₂-O-(CH₂)₂-, -CH₂-CH(-Y)-CH₂-, cyclohexan-1,2-diyl, cyclohexan-1,3-diyl oder cyclohexan-1,4-diyl steht,
R₃ für (-CH₂)₂CH-, (-CH₂)₃C-CH₃ oder (-CH₂)₃C-CH₂-CH₃ steht,
wobei Y = -H, -CH₃, -C₂H₅, -C₃H₇ oder -C₄H₉ ist und
m, n, o, p, q und r unabhängig voneinander eine ganze Zahl von 2 bis 18 bedeuten.

Besonders bevorzugt ist es dabei, daß R -CH(CH₃)-, R₁ -CH(CH₃)-COOY, mit Y = -C₂H₅, m, n, o, p, q oder r eine ganze Zahl von 2 bis 4 bedeutet.

Bei der Herstellung der Implantate werden daher Poly(hydroxyester) wie beispielsweise Poly-( L-lactid)e, Poly-(D,L-lactid)e, Polyglycolide, Poly-(caprolacton)e, Poly-(dioxanon)e, Poly-(hydroxybutter-säure)n, Poly-(hydroxyvaleriansäure)n, Poly-(glycosalicylat)e und Copolymere dieser Verbindungen eingesetzt. Besonders bevorzugt sind Poly-(hydroxyester), die durch Ringöffnungspolymerisation von Lactonen in Gegenwart eines biokompatiblen Startmoleküls hergestellt werden. Bevorzugte Lactone für die Durchführung der Ringöffnungspolymerisation sind beispielsweise L-Lactid, D,L-Lactid, Glycolid, p-Dioxanon und e-Caprolacton. Geeignete biokompatible Startmoleküle sind vorzugsweise aliphatische oder cycloaliphatische Verbindungen, die eine oder mehrere freie Hydroxylgruppen enthalten. Besonders geeignete Startmoleküle sind beispielsweise L-Milchsäure-alkylester, Cholesterol, Propan-1,2-diol, Triethylenglykol, Glycerol oder Pentaerythrit.

Es ist ferner erfindungsgemäß bevorzugt, daß das Verhältnis zwischen den festen höhermolekularen Polymeren und den flüssigen niedermolekularen Oligomeren 1:100 bis 1:1, insbesondere bevorzugt 1:10 bis 1:2 beträgt.

Die erfindungsgemäße pharmazeutische Zusammensetzung ist ferner dadurch gekennzeichnet, daß der biologisch aktive Wirkstoff aus der Gruppe der Hormone, Immunmodulatoren, Immunsuppressiva, Antibiotika, Zytostatika, Diuretika, Magen-Darm-Mittel, Herz-Kreislauf-Mittel und Neuropharmaka ausgewählt ist.

Dabei ist es erfindungsgemäß bevorzugt, daß der biologisch aktive Wirkstoff in der Hilfsstoffkombination in gelöster oder suspendierter Form vorliegt.

Erfindungsgemäß liegt die pharmazeutische Zusammensetzung in Form eines injizierbaren Mittels vor, welche nach Injektion unter dem Einfluß der Körperflüssigkeit ein Koagulat bildet.

Gegenstand der vorliegenden Erfindung ist ferner ein injizierbares Implantat, erhältlich durch Injektion einer erfindungsgemäßen pharmazeutischen Zusammensetzung in einen Körper.

Gegenstand der vorliegenden Erfindung ist auch ein Verfahren zur Herstellung eines injizierberen Implantats, dadurch gekennzeichnet, daß man eine erfindungsgemäße pharmazeutische Zusammensetzung in einen Körper eines Säugers injiziert.

Mit anderen Worten gesagt, wird die Aufgabe erfindungsgemäß dadurch gelöst, daß ein in situ Implantat, herstellbar durch Plazieren einer sterilen, injizierbaren Zusammensetzung aus einem bioabbaubaren Polymer einer Hydroxycarbonsäure, einem bioabbaubaren Oligomer einer Hydroxycarbonsäure und dem biologisch aktiven Stoff im Organismus, und Koagulation desselben unter dem Einfluß der Körperflüssigkeit, zur Verfügung gestellt wird.

Die vorliegende Erfindung betrifft somit biologisch abbaubare Zusammensetzungen oligomerer und polymerer Ester von Hydroxycarbonsauren, die durch geeignete Wahl der oligomeren und polymeren Komponenten als homogene Lösungen einstellbarer Viskosität oder niedrigschmelzende Festkörper ohne die Verwendung weiterer Lösungsmittel oder Weichmacher herstellbar sind. Die genannten Oligomer-Polymer-Zusammensetzungen sind in der Lage nach Injizierung in den menschlichen oder tierischen Organismus unter dem Einfluß von Körperflüssigkeit in situ Koagulate auszubilden. Die auf diese Weise gebildeten in situ Implantate können zur Applikation biologisch aktiver Stoffe im Organismus verwendet werden.

Bei einer Untersuchung einer großen Zahl von biokompatiblen Lösemitteln bzw. Komplexbildern wurde überraschenderweise gefunden, daß Oligomere verschiedener Hydroxycarbonsäuren mit definierten Strukturen in einem weiten Konzentrationsbereich in der Lage sind, bioabbaubare Polymere, insbesondere solche der Gruppe der Poly(hydroxyester) und deren Copolymere zu lösen bzw. in lösliche Komplexe zu überführen. Diese Lösungen bzw. löslichen Komplexe können in steriler Form parenteral appliziert werden und bilden unter dem Einfluß von Körperflüssigkeit in situ Koagulate aus.

Bevorzugt sind ferner Implantate, bei denen das bioabbaubare Oligomer eine Verbindung der allgemeinen Formel I, II oder III 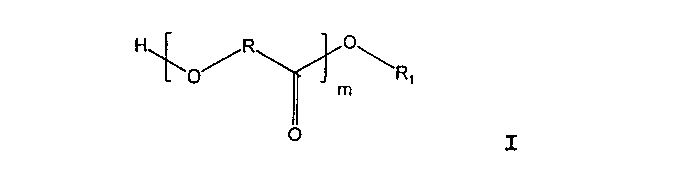 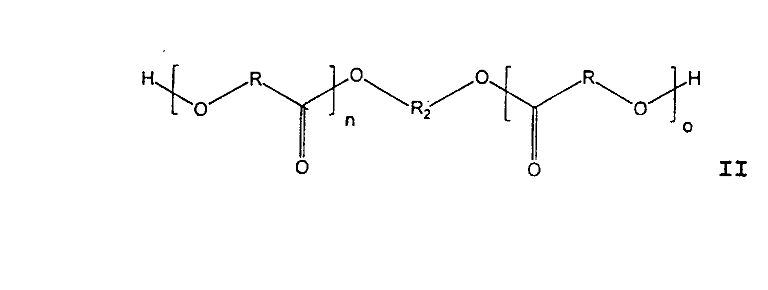 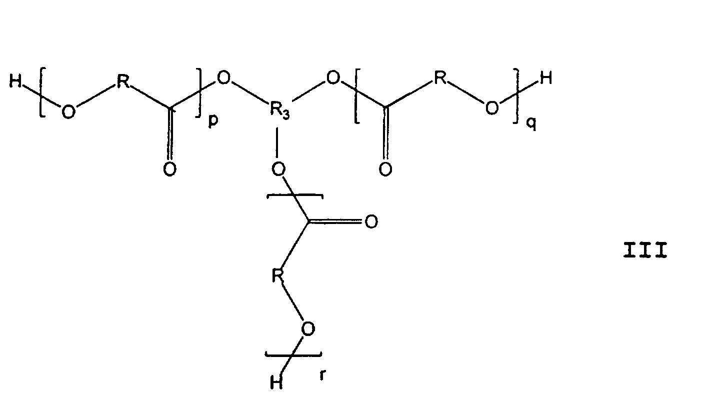 ist, worin
R für die Variablen m, n, o ,p, q und r jeweils gleich oder unterschiedlich ist und für -CH₂-, -CH(CH₃)-, -(CH₂)₅-, -CH₂-CH₂-O-CH₂-, -CH₂-CH₂-O-CH₂-CH₂-O-CH₂- oder deren Homologe mit jeweils bis zu 5 weiteren C-Atomen steht,
R₁ für -CH₂-COOY, -CH(CH₃)-COOY, -CH₂-CH₂-COOY, -CH₂-CH₂-CH₂-COOY, -CH₂-CH₂-CH₂-CH₂-COOY, -CH₂-CH₂-CH₂-CH₂-CH₂-COOY, -CH₂-CH(CH₃)-Y, -(cyclo-C₆H₁₁) oder -CH₂-C₆H₅- steht,
R₂ für -CH₂-CH(CH₃)-, -CH₂-CH₂-CH₂-, -CH₂-CH₂-CH₂-CH₂-, -CH₂-CH₂-CH₂-CH₂-CH₂-,- (CH₂)₂-O-(CH₂)₂-O-(CH₂)₂-, - (CH₂)₂-O- (CH₂)₂-O- (CH₂)₂-O- (CH₂)₂-, - (CH₂)₂-O- (CH₂)₂-O- (CH₂)₂-O- (CH₂)₂-O- (CH₂)₂-, -CH₂-CH(-Y)-CH₂-, cyclohexan-1,2-diyl, cyclohexan-1,3-diyl oder cyclohexan-1,4-diyl steht,
R₃ für (-CH₂)₂CH-, (-CH₂)₃C-CH₃ oder (-CH₂)₃C-CH₂-CH₃ steht,
wobei Y =-H, -CH₃, -C₂H₅, -C₃H₇ oder -C₄H₉ ist und
m, n, o, p, q und r unabhängig voneinander eine ganze Zahl von 2 bis 18 bedeuten.

Besonders bevorzugt ist es dabei, daß R -CH(CH₃)-, R₁ -CH(CH₃)-COOY, mit Y = -C₂H₅, m, n, o, p, q oder r eine ganze Zahl von 2 bis 4 bedeutet.

Bevorzugte Implantate weisen ein Verhältnis von bioabbaubarem Polymer zu Oligomer zwischen 1:100 und 1:1, insbesondere bevorzugt zwischen 1:6 und 1:2 auf.

Die erfindungsgemäßen Zusammensetzungen enthalten somit nur die oben genannten biokompatiblen, bioabbaubaren Polymere und Oligomere und kommen ohne zusätzliche Lösungsmittel oder Katalysatoren zur Anwendung.

Die erfindungsgemäßen Zusammensetzungen können biologisch aktive Stoffe enthalten. Bei bevorzugten Implantaten enthält das Koagulat mindestens einen biologisch aktiven Stoff, beispielsweise aus der Gruppe der Hormone, Immunmodulatoren, Immunsupressiva, Antibiotika, Zytostatika, Diuretika, Magen-Darm-Mittel, Herz-Kreislauf-Mittel, Anti inflammatoria, Analgetika, Lokalanaesthetika und/oder Neuropharmaka.

Die erfindungsgemäßen Zusammensetzungen sind soweit fließbar, daß sie weitgehend schmerzfrei injiziert werden können. Sie sind herkömmlichen Sterilisationsverfahren zugänglich.

Nach der Plazierung der erfindungsgemäßen, die biologisch aktive Substanz enthaltenden Zusammensetzungen im Organismus bildet sich unter dem Einfluß der Körperflüssigkeit ein Koagulat. Einherschreitend mit dem biologischen Abbau dieses Koagulats, der zwischen Wochen und Monaten bis Jahren in Abhängigkeit von der Zusammensetzung der Zusammensetzung betragen kann, wird die biologisch aktive Substanz freigesetzt.

Es ist daher bevorzugt, daß man die Freisetzung der biologisch aktiven Substanz durch die Bestandteile der sterilen, spritzbaren Zusammensetzung und deren Verhältnissen zueinander in der Lösung steuert. Auf diese Weise ist es möglich, die Freisetzungsgeschwindigkeit den pharmakokinetischen und pharmakodynamischen Eigenschaften der Wirkstoffe im Organismus anzupassen.

Gegenstand der vorliegenden Erfindung ist ferner ein Verfahren zur Herstellung eines Implantats, welches erfindungsgemäß dadurch gekennzeichnet ist, daß man eine sterile, spritzbare Zusammensetzung aus einem biologisch abbaubaren Polymer einer Hydroxycarbonsäure und einem flüssigen bioabbaubaren Oligomer einer Hydroxycarbonsäure im Organismus plaziert, und denselben unter dem Einfluß der Körperflüssigkeit koaguliert.

Die Erfindung wird durch die nachfolgenden Beispiele erläutert.

### Darstellung bioabbaubarer flüssiger Oligomere (Beispiel 1 und 2)

### Beispiel 1

### L-(-)-Milchsäureethylester-oligo-D,L-lactid

### (Ausgangsstoff 1)

Unter einem Stickstoffstrom wird ein Gemisch aus 15,0 g (104 mmol) D,L-Lactid, 12,294 g (104 mmol) L-(-)-Milchsäureethylester sowie 2 Tropfen Zinn(II)-2-ethylhexanoat bei 140 °C vier Stunden lang unter Rühren erhitzt. Nach Abkühlen der Schmelze auf Raumtemperatur wird das Reaktionsgemisch in 40 ml Methylenchlorid gelöst. Diese Lösung tropft man in 400 ml Heptan. Das Produkt scheidet sich als viskoses Öl ab. Man trennt die spezifisch leichtere Heptanphase ab, die im Produkt verbliebenen Lösemittelreste werden unter vermindertem Druck entfernt. Anschließend wird das Produkt im Vakuum bis zur Gewichtskonstanz getrocknet. Man erhält ein viskoses farbloses Öl.
Ausbeute: 16,02g
Mₙ(VPO): 354 g/mol

### Beispiel 2

### L-(-)-Milchsäureethylester-oligo-L-lactid

### (Ausgangsstoff 2)

Unter einem Stickstoffstrom wird ein Gemisch aus 15,0 g (104 mmol) L-Lactid, 12,294 g (104 mmol) L-(-)-Milchsäureethylester sowie 2 Tropfen Zinn (II)-2-ethylhexanoat, bei 140 °C vier Stunden lang unter Rühren erhitzt. Nach Abkühlen der Schmelze auf Raumtemperatur wird das Reaktionsgemisch in 40 ml Methylenchlorid gelöst. Diese Lösung tropft man in 400 ml Heptan. Das Produkt scheidet sich als viskoses Öl ab. Man trennt die spezifisch leichtere Heptanphase ab, die im Produkt verbliebenen Lösemittelreste werden unter vermindertem Druck entfernt. Anschließend wird das Produkt im Vakuum bis zur Gewichtskonstanz getrocknet. Man erhält ein viskoses farbloses Öl.
Ausbeute: 19,22 g
Mₙ (VPO): 362 g/mol

### Darstellung bioabbaubarer Polymere (Beispiele 3 bis 9)

### Beispiel 3

### (Ausgangsstoff 3)

Unter einem Stickstoffstrom wird ein Gemisch aus 13,0 g (90,2 mmol) D,L-Lactid, 0,532 g (4,5 mmol) L-(-)-Milchsäureethylester sowie 2 Tropfen Zinn (II)-2-ethylhexanoat, bei 140 °C über Nacht unter Rühren erhitzt. Nach Abkühlen der Schmelze auf Raumtemperatur wird das Reaktionsgemisch in 40 ml Methylenchlorid gelöst. Diese Lösung tropft man in 400 ml Heptan. Das Produkt fällt als klebriger Feststoff aus. Anschließend trennt man die spezifisch leichtere Heptanphase ab. Das Produkt wird in 40 ml Methylenchlorid aufgenommen. Nach Einengen des Lösungsmittels Unter vermindertem Druck, werden noch verbliebene Lösungsmittelrückstände durch mehrtägige Trocknung im Vakuum entfernt. Man erhält einen glasartigen transparenten Feststoff.
Ausbeute: 12,450 g
Mₙ (GPC,RI): 4346 g/mol

### Beispiel 4

### (Ausgangsstoff 4)

Unter einem Stickstoffstrom wird ein Gemisch aus 13,0 g (90,2 mmol) D,L-Lactid, 0,205 g (1,73 mmol) L-(-)-Milchsäureethylester sowie 2 Tropfen Zinn (II)-2-ethylhexanoat, bei 140 °C über Nacht unter Rühren erhitzt. Nach Abkühlen der Schmelze auf Raumtemperatur wird das Reaktionsgemisch in 40 ml Methylenchlorid gelöst. Diese Lösung tropft man in 400 ml Heptan. Das Produkt fällt als klebriger Feststoff aus. Anschließend trennt man die spezifisch leichtere Heptanphase ab. Das Produkt wird in 40 ml Methylenchlorid aufgenommen. Nach Einengen des Lösungsmittels unter vermindertem Druck, werden noch verbliebene Lösungsmittelrückstände durch mehrtägige Trocknung im Vakuum entfernt. Man erhält einen glasartigen transparenten Feststoff.
Ausbeute: 9,580 g
Mₙ (GPC,RI): 7790 g/mol

### Beispiel 5

### (Ausgangsstoff 5)

Unter einem Stickstoffstrom wird ein Gemisch aus 13,0 g (90,2 mmol) D,L-Lactid, 1,835 g (4,5 mmol) Cholesterol sowie 2 Tropfen Zinn(II)-2-ethylhexanoat, bei 140 °C über Nacht unter Rühren erhitzt. Nach Abkühlen der Schmelze auf Raumtemperatur wird das Reaktionsgemisch in 40 ml Methylenchlorid gelöst. Diese Lösung tropft man in 400 ml Heptan. Das Produkt fällt als klebriger Feststoff aus. Anschließend trennt man die spezifisch leichtere Heptanphase ab. Das Produkt wird in 40 ml Methylenchlorid aufgenommen. Nach Einengen des Lösungsmittels unter vermindertem Druck, werden noch verbliebene Lösungsmittelrückstände durch mehrtägige Trocknung im Vakuum entfernt. Man erhält einen glasartigen transparenten Feststoff.
Ausbeute: 13,905 g
Mₙ (GPC,RI): 4116 g/mol

### Beispiel 6

### (Ausgangsstoff 6)

Unter einem Stickstoffstrom wird ein Gemisch aus 13,0 g (90,2 mmol) D,L-Lactid, 0,918 g (2,25 mmol) Cholesterol sowie 2 Tropfen Zinn(II)-2-ethylhexanoat, bei 140 °C über Nacht unter Rühren erhitzt. Nach Abkühlen der Schmelze auf Raumtemperatur wird das Reaktionsgemisch in 40 ml Methylenchlorid gelöst. Diese Lösung tropft man in 400 ml Heptan. Das Produkt fällt als klebriger Feststoff aus. Anschließend trennt man die spezifisch leichtere Heptanphase ab. Das Produkt wird in 40 ml Methylenchlorid aufgenommen. Nach Einengen des Lösungsmittels unter vermindertem Druck, werden noch verbliebene Lösungsmittelrückstände durch mehrtägige Trocknung im Vakuum entfernt. Man erhält einen glasartigen transparenten Feststoff.
Ausbeute: 13,528 g
Mₙ (GPC,RI): 8682 g/mol

### Beispiel 7

### (Ausgangsstoff 7)

Unter einem Stickstoffstrom wird ein Gemisch aus 13,0 g (90,2 mmol) D,L-Lactid, 0,343 g (4,5 mmol) Propan-1,2-diol sowie 2 Tropfen Zinn(II)-2-ethylhexanoat, bei 140 °C über Nacht unter Rühren erhitzt. Nach Abkühlen der Schmelze auf Raumtemperatur wird das Reaktionsgemisch in 40 ml Methylenchlorid gelöst. Diese Lösung tropft man in 400 ml Heptan. Das Produkt fällt als klebriger Feststoff aus. Anschließend trennt man die spezifisch leichtere Heptanphase ab. Das Produkt wird in 40 ml Methylenchlorid aufgenommen. Nach Einengen des Lösungsmittels unter vermindertem Druck, werden noch verbliebene Lösungsmittelrückstände durch mehrtägige Trocknung im Vakuum entfernt. Man erhält einen glasartigen transparenten Feststoff.
Ausbeute: 12,130 g
Mₙ (GPC,RI): 3794 g/mol

### Beispiel 8

### (Ausgangsstoff 8)

Unter einem Stickstoffstrom wird ein Gemisch aus 13,0 g (90,2 mmol) D,L-Lactid, 0,171 g (2,25 mmol) Propan-1,2-diol sowie 2 Tropfen Zinn(II)-2-ethylhexanoat, bei 140 °C über Nacht unter Rühren erhitzt. Nach Abkühlen der Schmelze auf Raumtemperatur wird das Reaktionsgemisch in 40 ml Methylenchlorid gelöst. Diese Lösung tropft man in 400 ml Heptan. Das Produkt fällt als klebriger Feststoff aus. Anschließend trennt man die spezifisch leichtere Heptanphase ab. Das Produkt wird in 40 ml Methylenchlorid aufgenommen. Nach Einengen des Lösungsmittels unter vermindertem Druck, werden noch verbliebene Lösungsmittelrückstände durch mehrtägige Trocknung im Vakuum entfernt. Man erhält einen glasartigen transparenten Feststoff.
Ausbeute: 12,680 g
Mₙ (GPC,RI): 7784 g/mol

### Beispiel 9

### (Ausgangsstoff 9)

Unter einem Stickstoffstrom wird ein Gemisch aus 13,0 g (90,2 mmol) D,L-Lactid, 0,201 g (1,5 mmol) 1,1,1-Tris-(hydroxymethyl)propan sowie 2 Tropfen Zinn(II)-2-ethylhexanoat, bei 140 °C über Nacht unter Rühren erhitzt. Nach Abkühlen der Schmelze auf Raumtemperatur wird das Reaktionsgemisch in 40 ml Methylenchlorid gelöst. Diese Lösung tropft man in 400 ml Heptan. Das Produkt fällt als klebriger Feststoff aus. Anschließend trennt man die spezifisch leichtere Heptanphase ab. Das Produkt wird in 40 ml Methylenchlorid aufgenommen. Nach Einengen des Lösungsmittels unter vermindertem Druck, werden noch verbliebene Lösungsmittelrückstände durch mehrtägige Trocknung im Vakuum entfernt. Man erhält einen glasartigen transparenten Feststoff.
Ausbeute: 12,12 g
Mₙ (GPC,RI): 11053 g/mol

### Polymer-Oligomer-zusammensetzungen

### Beispiel 10

Ein Gemisch aus 100 mg Poly-D,L-lactid (Beispiel 5, JP 37) und 900 mg Oligoester (Beispiel 1, JP 43) werden 5 min lang bei 140 °C gerührt. Es entsteht eine viskose transparente Flüssigkeit. Beim Eintropfen dieser Polymer-Oligoester-Zusammensetzung in Wasser bildet sich ein formstabiles Koagulat aus.

### Freisetzung von biologisch aktiven Stoffen

### Beispiel 11

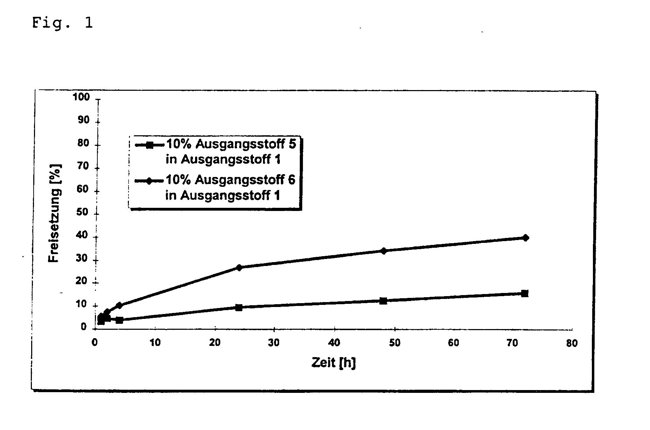

Die viskose transparente Lösung eines Gemisches (nach Beispiel 10) aus 100 mg Poly-D,L-lactid (Ausgangsstoff 5 oder 6) und 900 mg Oligomer (Ausgangsstoff 1) wird mit 6 mg Cytochrom C versetzt, welches suspendiert in dem Gemisch vorliegt. Die Suspension wird in eine Membran eingespritzt, die sich in einem Becherglas befindet, das 500 ml isotonische NaCl-Lösung enthält. Das Akzeptormedium wird gerührt und die Freisetzung von Cytochrom C aus dem in situ gebildeten Koagulat nach definierten Zeitintervallen ermittelt. Das Freisetzungsprofil ist in Fig. 1 wiedergegeben.

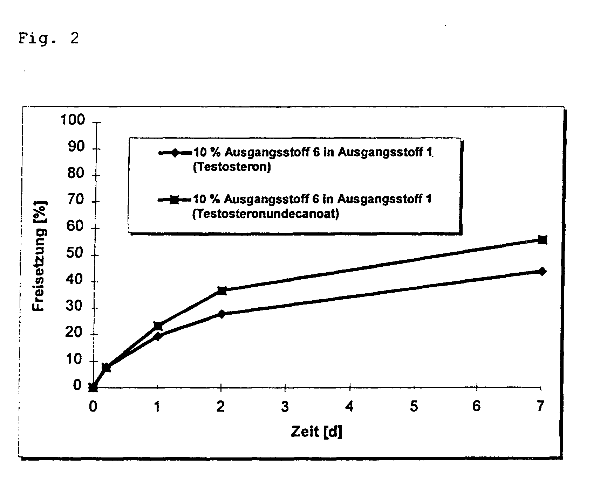

Die viskose transparente Lösung eines Gemisches (nach Beispiel 10) aus 100 mg Poly-D,L-lactid (Ausgangsstoff 6) und 900 mg Oligomer (Ausgangsstoff 1) wird mit 6 mg Testosteron bzw. Testosteronundecanoat versetzt, welches suspendiert in dem Gemisch vorliegt. Die Suspension wird in eine Membran eingespritzt, die sich in einem Becherglas befindet, das 500 ml isotonische NaCl-Lösung enthält. Das Akzeptormedium wird gerührt und die Freisetzung von Testosteron bzw. Testosteronundecanoat C aus dem in situ gebildeten Koagulat nach definierten Zeitintervallen ermittelt. Das Freisetzungsprofil ist in Fig. 2 wiedergegeben.

## Patentansprüche

1. Injizierbare Oligomer-Polymer-Zusammensetzung bestehend aus einer Kombination von mindestens zwei biologisch abbaubaren Hilfsstoffen und mindestens einem biologisch aktiven Wirkstoff wobei die biologisch abbaubaren Hilfsstoffe oligomere und polymere Ester von Hydroxycarbonsäuren sind.

2. Oligomer-Polymer-Zusammensetzung gemäß Anspruch 1, **dadurch gekennzeichnet, daß** die biologisch abbaubaren Hilfsstoffe Polymerisationsprodukte von gleichen oder unterschiedlichen Hydroxycarbonsäuren sind.

3. Oligomer-Polymer-Zusammensetzung gemäß Anspruch 2, **dadurch gekennzeichnet, daß** die Hydroxycarbonsäuren Milchsäure oder Glycolsäure sind.

4. Oligomer-Polymer-Zusammensetzung gemäß einem der voranstehenden Ansprüche, **dadurch gekennzeichnet, daß** jeweils mindestens einer der biologisch abbaubaren Hilfsstoffe ein flüssiges niedermolekulares Oligomer und der andere ein festes höhermolekulares Polymer ist.

5. oligomer-Polymer-Zusammensetzung gemäß Anspruch 4, **dadurch gekennzeichnet, daß** das flüssige niedermolekulare Oligomer eine Verbindung der allgemeinen Formel I, II oder III 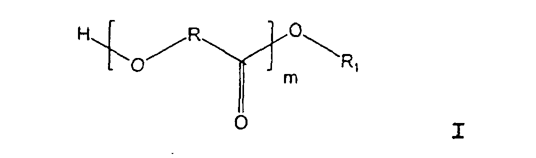 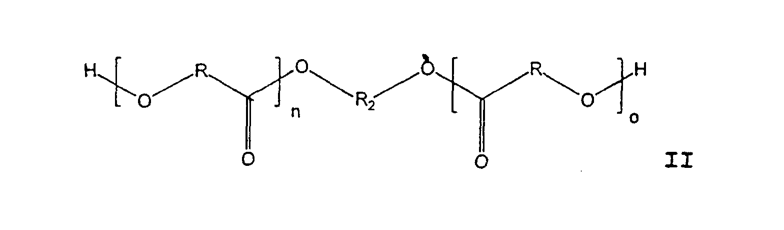 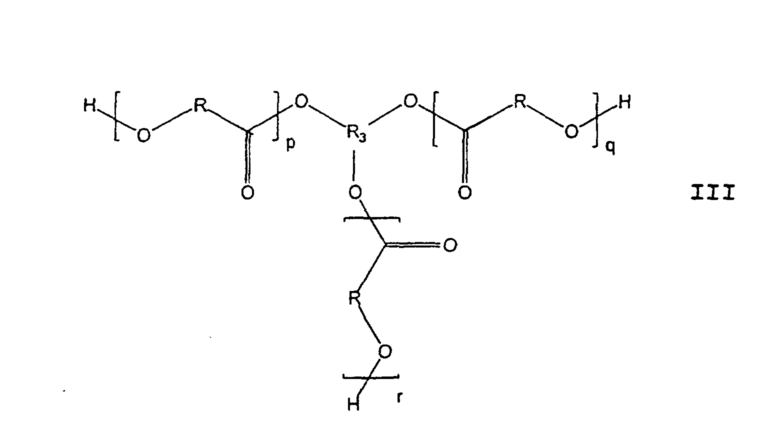 ist, worin
R für die Variablen m, n, o ,p, q und r jeweils gleich oder unterschiedlich ist und für -CH₂-, -CH(CH₃)-, -(CH₂)₅-, -CH₂-CH₂-O-CH₂-, -CH₂-CH₂-O-CH₂-CH₂-O-CH₂- oder deren Homologe mit jeweils bis zu 5 weiteren C-Atomen steht,
R₁ für -CH₂-COOY, -CH(CH₃)-COOY, -CH₂-CH₂-COOY, -CH₂-CH₂-CH₂-COOY, -CH₂-CH₂-CH₂-CH₂-COOY, -CH₂-CH₂-CH₂-CH₂-CH₂-COOY, -CH₂-CH(CH₃)-Y, -(cyclo-C₆H₁₁) oder -CH₂-C₆H₅- steht,
R₂ für -CH₂-CH(CH₃)-, -CH₂-CH₂-CH₂-, -CH₂-CH₂-CH₂-CH₂-, -CH₂-CH₂-CH₂-CH₂-CH₂-, -(CH₂)₂-O- (CH₂)₂-O- (CH₂)₂-, -(CH₂)₂-O-(CH₂)₂-O-(CH₂)₂-O- (CH₂)₂-, - (CH₂)₂-O-(CH₂)₂-O-(CH₂)₂-O- (CH₂)₂-O-(CH₂)₂-, -CH₂-CH(-Y)-CH₂-, cyclohexan-1,2-diyl, cyclohexan-1,3-diyl oder cyclohexan-1,4-diyl steht,
R₃ für (-CH₂)₂CH-, (-CH₂)₃C-CH₃ oder (-CH₂)₃C-CH₂-CH₃ steht,
wobei Y =-H, -CH₃, -C₂H₅, -C₃H₇ oder -C₄H₉ ist und
m, n, o, p, q und r unabhängig voneinander eine ganze Zahl von 2 bis 18 bedeuten.

6. Oligomer-Polymer-Zusammensetzung gemäß Anspruch 5, **dadurch gekennzeichnet, daß** R -CH(CH₃)-, R₁ -CH(CH₃)-COOY, mit Y = -C₂H₅, m, n, o, p, q oder r eine ganze Zahl von 2 bis 4 bedeuten.

7. Oligomer-Polymer-Zusammensetzung gemäß Anspruch 4, **dadurch gekennzeichnet, daß** das flüssige niedermolekulare Oligomer aus der folgenden Gruppe oder aus deren Mischungen ausgewählt ist, nämlich Poly(hydroxyester) wie Poly-( L-lactid)e, Poly-(D,Llactid)e, Polyglycolide, Poly-(caprolacton)e, Poly-(dioxanon)e, Poly-(hydroxybutter-säure)n, Poly-(hydroxyvaleriansäure) n, Poly- (glycosalicylat) e und Copolymere dieser Verbindungen, Paly-(hydroxyester), die durch Ringöffnungspolymerisation von Lactonen in Gegenwart eines biokompatiblen Startmoleküls herstellbar sind, nämlich L-Lactid, D,L-Lactid, Glycolid, p-Dioxanon und e-Caprolacton, mit aliphatischen oder cycloaliphatischen Verbindungen mit einer oder mehreren freie Hydroxylgruppen wie L-Milchsäure-alkylester, Cholesterol, Propan-1,2-diol, Triethylenglykol, Glycerol oder Pentaerythrit als biokompatible Startmoleküle.

8. Oligomer-Polymer-Zusammensetzung nach einem der Ansprüche 4 bis 7, **dadurch gekennzeichnet, daß** das Verhältnis zwischen den festen höhermolekularen Polymeren und den flüssigen niedermolekularen Oligomeren 1:100 bis 1:1, vorzugsweise 1:10 bis 1:2 beträgt.

9. Oligomer-Polymer-Zusammensetzung gemäß einem der voranstehenden Ansprüche, **dadurch gekennzeichnet, daß** der biologisch aktive Wirkstoff aus der Gruppe der Hormone, Immunmodulatoren, Immunsuppressiva, Antibiotika, Zytostatika, Diuretika, Magen-Darm-Mittel, Herz-Kreislauf-Mittel und Neuropharmaka ausgewählt ist.

10. Oligomer-Polymer-Zusammensetzung gemäß Anspruch 9, **dadurch gekennzeichnet, daß** der biologisch aktive Wirkstoff in der Hilfsstoffzusammensetzung in gelöster oder suspendierter Form vorliegt.

11. Oligomer-Polymer-Zusammensetzung gemäß einem der voranstehenden Ansprüche, **dadurch gekennzeichnet, daß** diese in Form eines injizierbaren Mittels vorliegt, welches nach Injektion unter dem Einfluß der Körperflüssigkeit eine Koagulat bildet.

12. Injizierbares Implantat, erhältlich durch Injektion einer Oligomer-Polymer-Zusammensetzung gemäß Anspruch 1 in einen Körper.

## Claims

1. Injectable oligomer-polymer composition consisting of a combination of at least two biodegradable excipients and at least one bioactive substance, where the biodegradable excipients are oligomeric and polymeric esters of hydroxy carboxylic acids.

2. Oligomer-polymer composition according to Claim 1, **characterized in that** the biodegradable excipients are polymerization products of identical or different hydroxy carboxylic acids.

3. Oligomer-polymer composition according to Claim 2, **characterized in that** the hydroxy carboxylic acids are lactic acid or glycolic acid.

4. Oligomer-polymer composition according to any of the preceding claims, **characterized in that** in each case at least one of the biodegradable excipients is a liquid low molecular weight oligomer and the other is a solid high molecular weight polymer.

5. Oligomer-polymer composition according to Claim 4, **characterized in that** the liquid low molecular weight oligomer is a compound of the general formula I, II or III 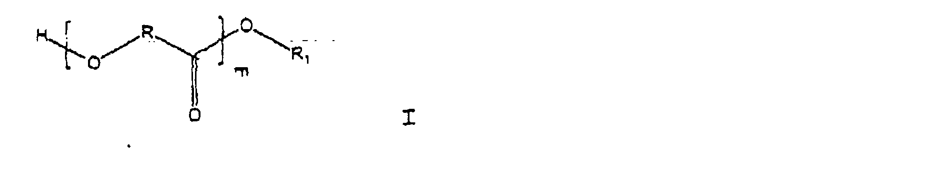 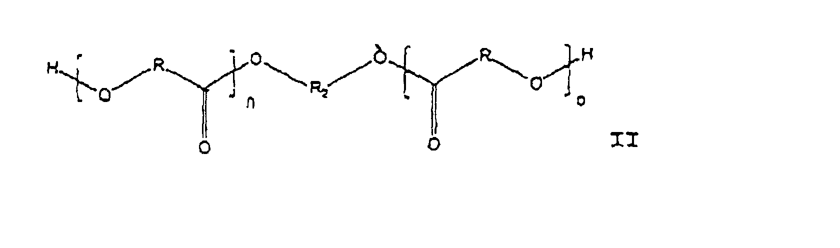 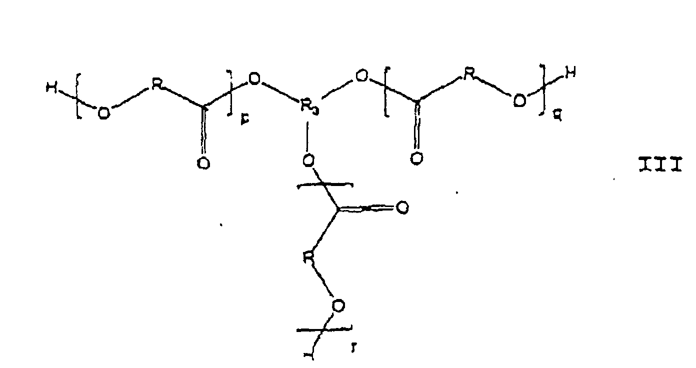 in which
R for the variables m, n, o, p, q and r is in each case identical or different and represents -CH₂-, -CH(CH₃)-, - (CH₂)₅-, -CH₂-CH₂-O-CH₂-, -CH₂-CH₂-O-CH₂-CH₂-O-CH₂- or homologues thereof with in each case up to 5 further C atoms,
R₁ represents -CH₂-COOY, -CH(CH₃)-COOY, -CH₂-CH₂-COOY, -CH₂-CH₂-CH₂-COOY, -CH₂-CH₂-CH₂-CH₂-COOY, -CH₂-CH₂-CH₂-CH₂-CH₂-COOY, -CH₂-CH (CH₃)-Y, -(cyclo-C₆H₁₁) or -CH₂-C₆H₅-,
R₂ represents -CH₂-CH(CH₃)-, -CH₂-CH₂-CH₂-, -CH₂-CH₂-CH₂-CH₂-, -CH₂-CH₂-CH₂-CH₂-CH₂-, -(CH₂)₂-O-(CH₂)₂-O- (CH₂)₂-, - (CH₂)₂-O-(CH₂)₂-O-(CH₂)₂-O-(CH₂)₂-, - (CH₂)₂-O-(CH₂)₂-O-(CH₂)₂-O-(CH₂)₂-O-(CH₂)₂-, -CH₂-CH(-Y)-CH₂-, cyclohexane-1,2-diyl, cyclohexane-1,3-diyl or cyclohexane-1,4-diyl,
R₃ represents (-CH₂)₂CH-, (-CH₂)₃C-CH₃ or (-CH₂)₃C-CH₂-CH₃, where Y is -H, -CH₃, -C₂H₅, -C₃H₇ or -C₄H₉, and
m, n, o, p, q and r denote, independently of one another, an integer from 2 to 18.

6. Oligomer-polymer composition according to Claim 5, **characterized in that** R denotes -CH(CH₃)-, R₁ denotes -CH(CH₃)-COOY with Y = -C₂H₅, m, n, o, p, q or r denotes an integer from 2 to 4.

7. Oligomer-polymer composition according to Claim 4, **characterized in that** the liquid low molecular weight oligomer is selected from the following group or from mixtures thereof, namely poly(hydroxy esters) such as poly(L-lactide)s, poly(D,L-lactide)s, polyglycolides, poly(caprolactone)s, poly(dioxanone)s, poly(hydroxybutyric acid)s, poly(hydroxyvaleric acid)s, poly(glycosalicylate)s and copolymers of these compounds, poly(hydroxy esters) which can be prepared by ring-opening polymerization of lactones in the presence of a biocompatible starter molecule, namely L-lactide, D,L-lactide, glycolide, p-dioxanone and e-caprolactone, with aliphatic or cycloaliphatic compounds having one or more free hydroxyl groups such as alkyl L-lactates, cholesterol, propane-1,2-diol, triethylene glycol, glycerol or pentaerythritol as biocompatible starter molecules.

8. Oligomer-polymer composition according to any of Claims 4 to 7, **characterized in that** the ratio between the solid high molecular weight polymers and the liquid low molecular weight oligomers is 1:100 to 1:1, preferably 1:10 to 1:2.

9. Oligomer-polymer composition according to any of the preceding claims, **characterized in that** the bioactive substance is selected from the group of hormones, immunomodulators, immunosuppressants, antibiotics, cytostatics, diuretics, gastrointestinal agents, cardiovascular agents and neuropharmaceuticals.

10. Oligomer-polymer composition according to Claim 9, **characterized in that** the bioactive substance is present in the excipient composition in dissolved or suspended form.

11. Oligomer-polymer composition according to any of the preceding claims, **characterized in that** the latter is in the form of an injectable composition which, after injection, forms a coagulum under the influence of body fluid.

12. Injectable implant obtainable by injection of an oligomer-polymer composition according to Claim 1 into a body.

## Revendications

1. Composition d'oligomère-polymère injectable constituée d'une combinaison d'au moins deux excipients biodégradables et d'au moins une substance biologiquement active, les excipients biodégradables étant des esters oligomères et polymères d'acides hydroxycarboxyliques.

2. Composition d'oligomère-polymère selon la revendication 1, **caractérisée en ce que** les excipients biodégradables sont des produits de polymérisation d'acides hydroxycarboxyliques identiques ou différents.

3. Composition d'oligomère-polymère selon la revendication 2, **caractérisée en ce que** les acides hydroxycarboxyliques sont l'acide lactique ou l'acide glycolique.

4. Composition d'oligomère-polymère selon l'une quelconque des revendications précédentes, **caractérisée en ce que**, dans chaque cas, au moins l'un des excipients biodégradables est un oligomère liquide à faible poids moléculaire et l'autre est un polymère solide à poids moléculaire élevé.

5. Composition d'oligomère-polymère selon la revendication 4, **caractérisée en ce que** l'oligomère liquide à faible poids moléculaire est un composé de formules générales I, II ou III 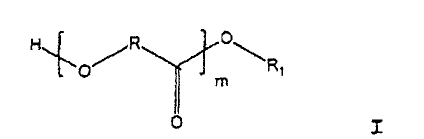 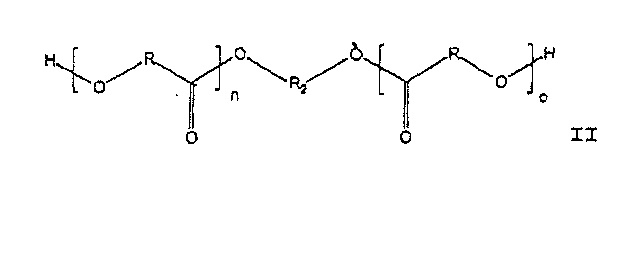 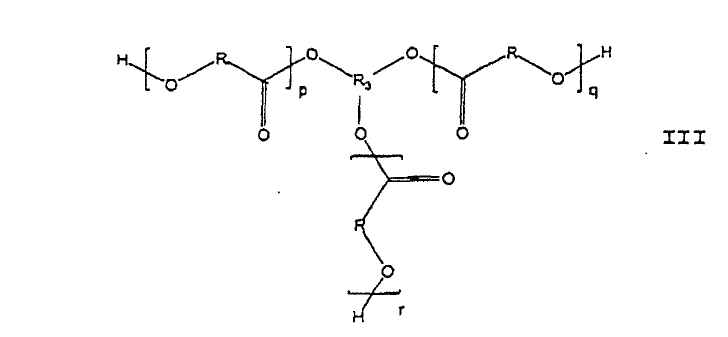 dans lesquelles
R pour les variables m, n, o, p, q et r est dans chaque cas identique ou différent et représente -CH₂-, -CH(CH₃)-, -(CH₂)₅-, -CH₂-CH₂-O-CH₂-, -CH₂-CH₂-O-CH₂-CH₂-O-CH₂- ou leurs homologues ayant dans chaque jusqu'à 5 atomes de carbone supplémentaires,
R₁ représente -CH₂-COOY, -CH(CH₃)-COOY, -CH₂-CH₂-COOY, -CH₂-CH₂-CH₂-COOY, -CH₂-CH₂-CH₂-CH₂-COOY, -CH₂-CH₂-CH₂-CH₂-CH₂-COOY, -CH₂-CH(CH₃)-Y, -(cyclo-C₆H₁₁) ou -CH₂-C₆H₅-,
R₂ représente -CH₂-CH(CH₃)-, -CH₂-CH₂-CH₂-, -CH₂-CH₂-CH₂-CH₂-, -CH₂-CH₂-CH₂-CH₂-CH₂-, -(CH₂)₂-O-(CH₂)₂-O-(CH₂)₂-, - (CH₂)₂-O-(CH₂)₂-O-(CH₂)₂-O-(CH₂)₂-, - (CH₂)₂-O-(CH₂)₂-O-(CH₂)₂-O-(CH₂)₂-O-(CH₂)₂-, -CH₂-CH(-Y)-CH₂-, un cyclohexane-1,2-diyle, un cyclohexane-1,3-diyle ou un cyclohexane-1,4-diyle,
R₃ représente (-CH₂)₂CH-, (-CH₂)₃C-CH₃ ou (-CH₂)₃C-CH₂-CH₃,
où Y représente -H, -CH₃, -C₂H₅, -C₃H₇ ou -C₄H₉, et
m, n, o, p, q et r représentent, indépendamment les uns des autres, un entier de 2 à 18.

6. Composition d'oligomère-polymère selon la revendication 5, **caractérisée en ce que** R représente -CH(CH₃)-, R₁ représente -CH(CH₃)-COOY où Y = -C₂H₅, m, n, o, p, q ou r représente un entier de 2 à 4.

7. Composition d'oligomère-polymère selon la revendication 4, **caractérisée en ce que** l'oligomère liquide à faible poids moléculaire est choisi parmi le groupe suivant ou parmi leurs mélanges, à savoir des polyhydroxyesters tels que des poly(L-lactides), des poly(D,L-lactides), des polyglycolides, des polycaprolactones, des polydioxanones, des polyacides hydroxybutyriques, des polyacides hydroxyvalériques, des polyglycosalicylates et des copolymères de ces composés, des polyhydroxyesters qui peuvent être préparés par polymérisation à décyclisation de lactones en présence d'une molécule d'amorçage biocompatible, à savoir le L-lactide, le D,L-lactide, le glycolide, la p-dioxanone et l'ε-caprolactone, avec des composés aliphatiques ou cycloaliphatiques ayant un ou plusieurs groupes hydroxy libres, tels que des L-lactates d'alkyle, le cholestérol, le propane-1,2-diol, le triéthylèneglycol, le glycérol ou le pentaérythritol en tant que molécules d'amorçage biocompatibles.

8. Composition d'oligomère-polymère selon l'une quelconque des revendications 4 à 7, **caractérisée en ce que** le rapport entre les polymères solides à poids moléculaire élevé et les oligomères liquides à faible poids moléculaire est de 1:100 à 1:1, de préférence de 1:10 à 1:2.

9. Composition d'oligomère-polymère selon l'une quelconque des revendications précédentes, **caractérisée en ce que** la substance biologiquement active est choisie parmi le groupe constitué des hormones, des immunomodulateurs, des immunosuppresseurs, des antibiotiques, des cytostatiques, des diurétiques, des agents gastro-intestinaux, des agents cardiovasculaires et des agents neuropharmaceutiques.

10. Composition d'oligomère-polymère selon la revendication 9, **caractérisée en ce que** la substance biologiquement active est présente dans la composition d'excipients sous forme dissoute ou en suspension.

11. Composition d'oligomère-polymère selon l'une quelconque des revendications précédentes, **caractérisée en ce que** celle-ci se présente sous la forme d'une composition injectable qui, après injection, forme un coagulat sous l'influence du fluide corporel.

12. Implant injectable pouvant être obtenu par injection d'une composition d'oligomère-polymère selon la revendication 1 dans un organisme.
